Europäisches Patentamt
European Patent Office
Office européen des brevets

(19)

(11) Veröffentlichungsnummer: **0 346 279 A1**

(12) **EUROPÄISCHE PATENTANMELDUNG**

(21) Anmeldenummer: **89810399.9**

(22) Anmeldetag: **29.05.89**

(51) Int. Cl.4: **C 08 K 5/07**
C 08 L 57/08, C 07 C 49/12

(30) Priorität: **06.06.88 CH 2139/88**

(43) Veröffentlichungstag der Anmeldung:
**13.12.89 Patentblatt 89/50**

(84) Benannte Vertragsstaaten: **BE DE FR GB IT**

(71) Anmelder: **CIBA-GEIGY AG**
**Klybeckstrasse 141**
**CH-4002 Basel (CH)**

(72) Erfinder: **Wirth, Hermann O., Dr.**
**Lessingstrasse 24**
**D-6140 Bensheim 3 (DE)**

**Friedrich, Hans-Helmut**
**Am Rauhenstein 8**
**D-6147 Lautertal 2 (DE)**

**Abeler, Gerd, Dr.**
**Alter Wixhäuser Weg 61**
**D-6100 Darmstadt (DE)**

(54) **Propan-1,3-dionderivate und deren Verwendung als Stabilisatoren für chlorhaltige Polymerisate.**

(57) Zusammensetzung enthaltend a) ein chlorhaltiges Polymerisat und b) mindestens eine Verbindung der Formel I,

$$R_1-\underset{\underset{O}{\|}}{C}-CH_2-\underset{\underset{O}{\|}}{C}-R_2 \quad (I)$$

worin $R_1$ $C_1$-$C_{10}$-Alkyl, $C_2$-$C_{10}$-Hydroxyalkyl, Phenyl, Hydroxyphenyl, $C_7$-$C_{10}$-Phenylalkyl oder am Phenylring durch eine OH-Gruppe substituiertes $C_7$-$C_{10}$-Phenylalkyl ist und $R_2$ $C_2$-$C_{10}$-Hydroxyalkyl, Hydroxyphenyl oder am Phenylring durch eine OH-Gruppe substituiertes $C_7$-$C_{10}$-Phenylalkyl darstellt, mit der Bedingung, dass $R_2$ verschieden von Hydroxyphenyl ist, wenn $R_1$ Phenyl oder Hydroxyphenyl bedeutet, und/oder mindestens eine Verbindung der Formel I, worin die in den Resten $R_1$ und $R_2$ enthaltenen OH Gruppen durch $C_2$-$C_{12}$-Alkanoyloxy ersetzt sind.

Die Verbindungen der Formel IA,

$$R-\underset{\underset{O}{\|}}{C}-CH_2-\underset{\underset{O}{\|}}{C}-(CH_2)_5-OH \quad (IA)$$

worin R $C_1$-$C_{10}$-Alkyl, $C_2$-$C_{10}$-Hydroxyalkyl, Phenyl, Hydroxyphenyl, $C_7$-$C_{10}$-Phenylalkyl oder am Phenylring durch eine OH-Gruppe substituiertes $C_7$-$C_{10}$-Phenylalkyl ist, sind neu.



Bundesdruckerei Berlin

**Beschreibung**

**Propan-1,3-dionderivate und deren Verwendung als Stabilisatoren für chlorhaltige Polymerisate**

Die vorliegende Erfindung betrifft mit Propan-1,3-dionderivaten gegen thermischen Abbau stabilisierte chlorhaltige Polymerisate, die Verwendung von Propan-1,3-dionderivaten als Stabilisatoren sowie neue Propan-1,3-dionderivate.

Es ist bekannt, dass chlorhaltige Polymerisate gegen den schädigenden Einfluss von Licht und Wärme, insbesondere bei der Verarbeitung zu Formteilen, geschützt werden müssen. 1,3-Diketone und deren Verwendung als Stabilisatoren werden beispielsweise in US-A-3,493,536, US-A-4,123,399, US-A-4,244,848, US-A-4,252,698, US-A-4,282,141, US-A-4,381,360, GB-A-788,428 und EP-A-35,268 offenbart.

Ferner wird die Herstellung von 1,3-Diketonen in einer Reihe von Veröffentlichungen ausführlich beschrieben. Als repräsentative Publikationen seien genannt: G.A. Kraus et al., J. Org. Chem. 49, 3212-3214 (1984), M.R. Detty, J. Org. Chem. 44, 2073-2077 (1979).

Die Verwendung von 1,3-Diketonen als Ausgangsprodukte für die Herstellung von Heterocyclen wird in folgenden Veröffentlichungen beschrieben: G.W. Cannon et al., J. Org. Chem. 17, 1245-1251 (1952), V. Koppe et al., Eur. J. Med. Chem.-Chimica Therapeutica 10, 154-161 (1975), Chemical Abstracts 71:49840d (1969), K. Uchino et al., Tetrahedron Letters 26, 1319-1320 (1985).

Die Behandlung von Metalloberflächen mit 1,3-Diketonen wird in US-A-3,615,888 beschrieben und US-A-3,816,615 offenbart Haarbleichmittel, die 1,3-Diketone enthalten.

Chemical Abstracts 87:135221q (1977) offenbart die Verbindung 7-Hydroxy-5, 5-dimethylheptan-2 4-dion.

Die vorliegende Erfindung betrifft Zusammensetzungen enthaltend a) ein chlorhaltiges Polymerisat und b) mindestens eine Verbindung der Formel I,

$$R_1-\underset{\underset{O}{\|}}{C}-CH_2-\underset{\underset{O}{\|}}{C}-R_2 \qquad (I)$$

worin $R_1$ $C_1$-$C_{10}$-Alkyl, $C_2$-$C_{10}$-Hydroxyalkyl, Phenyl, Hydroxyphenyl, $C_7$-$C_{10}$-Phenylalkyl oder am Phenylring durch eine OH-Gruppe substituiertes $C_7$-$C_{10}$-Phenylalkyl ist und $R_2$ $C_2$-$C_{10}$-Hydroxyalkyl, Hydroxyphenyl oder am Phenylring durch eine OH-Gruppe substituiertes $C_7$-$C_{10}$-Phenylalkyl darstellt, mit der Bedingung, dass $R_2$ verschieden von Hydroxyphenyl ist, wenn $R_1$ Phenyl oder Hydroxyphenyl bedeutet, und/oder mindestens eine Verbindung der Formel I, worin die in den Resten $R_1$ und $R_2$ enthaltenen OH Gruppen durch $C_2$-$C_{12}$-Alkanoyloxy ersetzt sind.

$R_1$ bedeutet als $C_1$-$C_{10}$-Alkyl zum Beispiel Methyl, Ethyl, n-Propyl, Isopropyl, n-Butyl, tert-Butyl, n-Pentyl, n-Hexyl, n-Heptyl, n-Octyl oder n-Decyl. $C_1$-$C_4$-Alkyl, insbesondere Methyl und tert-Butyl, ist bevorzugt.

Beispiele für $R_1$ und $R_2$ als $C_2$-$C_{10}$-Hydroxyalkyl sind 2-Hydroxyethyl, 3-Hydroxypropyl, 4-Hydroxybutyl, 2-Hydroxybutyl, 5-Hydroxypentyl, 1,1-Dimethyl-3-hydroxypropyl, 6-Hydroxyhexyl, 8-Hydroxyoctyl und 10-Hydroxydecyl. 2-Hydroxyethyl, 5-Hydroxypentyl, 6-Hydroxyhexyl und 7-Hydroxyheptyl sind bevorzugt. Vorzugsweise befindet sich die Hydroxyalkylgruppe nicht in 1-Stellung. $R_2$ bedeutet insbesondere eine Gruppe der Formel $-(CH_2)_n-OH$, worin n eine ganze Zahl von 2 bis 7 darstellt. Von besonderem Interesse ist $R_2$ als 5-Hydroxypentyl.

Bedeuten $R_1$ und $R_2$ Hydroxyphenyl, so kann sich die OH-Gruppe in ortho-, meta- oder para-Stellung befinden. o-Hydroxyphenyl ist bevorzugt.

Beispiele für $R_1$ als $C_7$-$C_{10}$-Phenylalkyl sind Benzyl und 2-Phenylethyl. $R_1$ und $R_2$ bedeuten als $C_7$-$C_{10}$-Phenylalkyl, welches am Phenylring durch eine OH-Gruppe substituiert ist, z.B. (2-Hydroxyphenyl)methyl, (3-Hydroxyphenyl)methyl, (4-Hydroxyphenyl)methyl, 2-(2-Hydroxyphenyl)ethyl und 2-(4-Hydroxyphenyl)ethyl.

$C_2$-$C_{12}$-Alkanoyloxy bedeutet zum Beispiel Acetyloxy, Propionyloxy, Butyryloxy, Pentanoyloxy, Hexanoyloxy, Heptanoyloxy, Octanoyloxy, Nonanoyloxy, Decanoyloxy, Undecanoyloxy oder Dodecanoyloxy.

Von Interesse sind Zusammensetzungen enthaltend als Komponente b) mindestens eine Verbindung der Formel 1, worin $R_1$ $C_1$-$C_{10}$-Alkyl, $C_2$-$C_{10}$-Hydroxyalkyl, Phenyl, Hydroxyphenyl, $C_7$-$C_{10}$-Phenylalkyl oder am Phenylring durch eine OH-Gruppe substituiertes $C_7$-$C_{10}$-Phenylalkyl ist und $R_2$ $C_2$-$C_{10}$-Hydroxyalkyl, Hydroxyphenyl oder am Phenylring durch eine OH-Gruppe substituiertes $C_7$-$C_{10}$-Phenylalkyl darstellt, mit der Bedingung, dass $R_2$ verschieden von Hydroxyphenyl ist, wenn $R_1$ Phenyl oder Hydroxyphenyl bedeutet.

Bevorzugt sind Zusammensetzungen enthaltend als Komponente b) mindestens eine Verbindung der Formel I, worin $R_1$ $C_1$-$C_{10}$-Alkyl, $C_2$-$C_{10}$-Hydroxyalkyl oder Phenyl bedeutet und $R_2$ $C_2$-$C_{10}$-Hydroxyalkyl oder Hydroxyphenyl ist.

Besonders bevorzugt sind Zusammensetzungen enthaltend als Komponente b) mindestens eine Verbindung der Formel I, worin $R_1$ $C_1$-$C_4$-Alkyl oder Phenyl bedeutet und $R_2$ $C_2$-$C_7$-Hydroxyalkyl oder Hydroxyphenyl ist.

Ebenfalls bevorzugt sind Zusammensetzungen enthaltend als Komponente b) mindestens eine Verbindung der Formel I, worin $R_1$ Methyl, Butyl oder Phenyl ist und $R_2$ 2-Hydroxyethyl, 5-Hydroxypentyl, 6-Hydroxyhexyl, 7-Hydroxyheptyl oder ortho-Hydroxyphenyl darstellt.

Von Interesse sind auch Zusammensetzungen, worin die Komponente b) 1-Phenyl-8-hydroxyoctan-1,3-dion, 1-t-Butyl-8-hydroxyoctan-1,3-dion oder 1-(o-Hydroxyphenyl)butan-1,3-dion, insbesondere 1-Phenyl-8-hydroxyoctan-1,3-dion, ist.

Bei den chlorhaltigen Polymerisaten handelt es sich bevorzugt um Vinylchloridhomopolymere oder

-copolymere. Als Comonomere für die Copolymerisate kommen z.B. in Frage: Vinylacetat, Vinylidenchlorid, Transdichlorethen, Ethylen, Propylen, Butylen, Maleinsäure, Acrylsäure, Fumarsäure, Itaconsäure. Weitere geeignete chlorhaltige Polymere sind nachchloriertes PVC und chlorierte Polyolefine, ferner Pfropfpolymerisate von PVC mit EVA, ABS und MBS. Bevorzugte Substrate sind auch Mischungen der vorstehend genannten Homo- und Copolymerisate, insbesondere Vinylchlorid-Homopolymerisate, mit anderen thermoplastischen oder/und elastomeren Polymeren, insbesondere mit ABS, MBS, NBR, SAN, EVA.

Weiterhin bevorzugt sind Suspensions- und Massepolymere sowie Emulsionspolymere.

Als chlorhaltiges Polymerisat ist Polyvinylchlorid besonders bevorzugt.

Es ist vorteilhaft, die Verbindungen der Formel I zusammen mit bekannten Thermostabilisatoren einzusetzen, wie z.B. Me(II)-Phenolaten, insbesondere $C_7$-$C_{20}$-Alkylphenolaten, beispielsweise Nonylphenolat, oder Me(II)-Carboxylaten. Me(II) bedeutet z.B. Ba, Ca, Mg, Cd oder Zn. Bei den Carboxylaten handelt es sich bevorzugt um Salze von Carbonsäuren mit 7 bis 20 C-Atomen, beispielsweise Benzoate, Alkenoate oder Alkanoate, bevorzugt Stearate, Oleate, Laurate, Palmitate, Hydroxystearate oder 2-Ethylhexanoate. Besonders bevorzugt sind Stearate, Oleate und p-tert-Butylbenzoate.

Bevorzugt werden die Verbindungen der Formel I auch zusammen mit Organozinnverbindungen, welche ebenfalls bekannte Thermostabilisatoren sind, eingesetzt. Beispiele für solche Organozinnverbindungen sind Organozinncarboxylate der unten angegebenen Formeln,

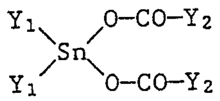 ,

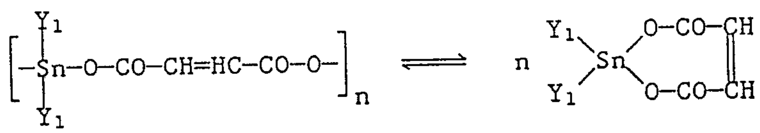 ,

worin die Reste $Y_1$ unabhängig voneinander $C_1$-$C_{12}$-Alkyl bedeuten und die Reste $Y_2$ unabhängig voneinander $C_1$-$C_{18}$-Alkyl oder eine Gruppe $-CH=CH-CO-O-(C_1$-$C_{18}$-Alkyl) darstellen.

Alkyl bedeutet beispielsweise Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Heptadecyl oder Octadecyl. $C_1$-$C_{10}$-Alkyl ist bevorzugt.

Eine besonders bevorzugte Organozinnverbindung ist

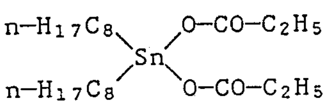 .

Zusätzlich können die chlorhaltigen Polymerisate in üblichen Mengen herkömmliche PVC-Stabilisatoren enthalten, wie beispielsweise Epoxiverbindungen, wie epoxidierte Oele und vorzugsweise epoxidierte Fettsäureester, insbesondere epoxidiertes Sojabohnenöl, sowie Phosphite, bevorzugt Triorganophosphite der Formel

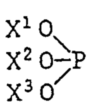 ,

worin $X^1$, $X^2$ und $X^3$ unabhängig voneinander $C_4$-$C_{18}$-Alkyl, Phenyl oder durch ein bis drei $C_1$-$C_{12}$-Alkylgruppen substituiertes Phenyl bedeuten.

Ein bevorzugter Gegenstand der Erfindung sind daher Zusammensetzungen enthaltend ausser den Komponenten a) und b) zusätzlich eine Epoxiverbindung und mindestens ein Me(II)-Carboxylat und/oder Me(II)-Phenolat, wobei Me(II) Ba, Ca, Mg, Cd oder Zn bedeutet und gegebenenfalls ein Phosphit.

Gemäss einer weiteren Bevorzugung enthalten die erfindungsgemässen Zusammensetzungen ausser den Komponenten a) und b) zusätzlich epoxidiertes Sojabohnenöl und mindestens ein Me(II)-Carboxylat, wobei Me(II) Ba, Ca, Mg oder Zn bedeutet. Gemische aus Ba/Zn- oder Ca/Zn-Carboxylaten werden dabei als Costabilisatoren besonders bevorzugt.

Ebenfalls ein bevorzugter Gegenstand der Erfindung sind Zusammensetzungen enthaltend ausser den Komponenten a) und b) zusätzlich eine Organozinnverbindung und gegebenenfalls ein Me(II)-Carboxylat, wobei Me(II) die oben angegebenen Bedeutungen besitzt und bevorzugt Zn darstellt.

Die bekannten Thermostabilisatoren (z.B. Carboxylate, Phenolate oder Organozinnverbindungen) können in dem zu stabilisierenden Material in einer dem Fachmann bekannten Konzentration vorliegen, wie zum Beispiel in Mengen von 0,05 bis 5 Gew.%.

Die Phosphite werden z.B. in Konzentrationen von 0,3 bis 5, vorzugsweise 0,5 bis 1 Gew.% und die Epoxiverbindungen, wie z.B. das epoxidierte Sojabohnenöl, in Konzentrationen von 1 bis 8, vorzugsweise 1

bis 3 Gew.% eingesetzt.

Die Verbindungen der Formel I werden beispielsweise in Mengen von 0,05 bis 1, bevorzugt 0,1 bis 0,5 Gew.% in das chlorhaltige Polymerisat eingearbeitet.

Die Angabe Gew.% bezieht sich jeweils auf das zu stabilisierende Material.

Je nach dem Verwendungszweck der Polymerisate können vor oder bei der Einarbeitung der Stabilisatoren auch weitere Zusätze eingearbeitet werden, wie zum Beispiel phenolische Antioxidantien, Gleitmittel (bevorzugt Montanwachse oder Glycerinester), Fettsäureester, Paraffine, Weichmacher, Füllstoffe, Russ, Asbest, Kaolin, Talk, Glasfasern, Modifikatoren (wie etwa Schlagzäh-Zusätze), optische Aufheller, Pigmente, Lichtschutzmittel, UV-Absorber, Flammschutzmittel oder Antistatika.

Die Einarbeitung der Stabilisatorkomponenten in das chlorhaltige Polymerisat erfolgt am günstigsten, wie üblich, auf einem 2-Walzenstuhl bei Temperaturen zwischen 150° und 200°C. Im allgemeinen lässt sich eine genügende Homogenisierung innerhalb von 5 bis 15 Minuten erreichen. Die Zugabe der Komponenten kann einzeln oder gemeinsam als Vorgemisch erfolgen. Als zweckmässig hat sich ein flüssiges Vorgemisch erwiesen, d.h. es wird in Gegenwart von indifferenten Lösungsmitteln und/oder Weichmachern gearbeitet.

Ein weiterer Gegenstand der Erfindung sind die neuen Verbindungen der Formel IA,

$$R-\underset{\underset{O}{\|}}{C}-CH_2-\underset{\underset{O}{\|}}{C}-(CH_2)_5-OH \quad (IA)$$

worin R $C_1$-$C_{10}$-Alkyl, $C_2$-$C_{10}$-Hydroxyalkyl, Phenyl, Hydroxyphenyl, $C_7$-$C_{10}$-Phenylalkyl oder am Phenylring durch eine OH-Gruppe substituiertes $C_7$-$C_{10}$-Phenylalkyl ist.

Beispiele für R sind die oben für $R_1$ angegebenen Definitionen.

R bedeutet bevorzugt $C_1$-$C_{10}$-Alkyl, Phenyl oder $C_7$-$C_{10}$-Phenylalkyl, insbesondere $C_1$-$C_4$-Alkyl oder Phenyl.

Besonders bevorzugte neue Verbindungen der Formel IA sind 1-Phenyl-8-hydroxyoctan-1,3-dion und 1-t-Butyl-8-hydroxyoctan-1,3-dion.

Die Verbindungen der Formeln I und IA können in Analogie zu bekannten Verfahren hergestellt werden, zum Beispiel durch Umsetzung einer Verbindung der Formel II

$$R_1-\underset{\underset{O}{\|}}{C}-CH_3 \quad (II)$$

mit einer Verbindung der Formel III,

$$R'-O-\underset{\underset{O}{\|}}{C}-R_2 \quad (III)$$

wobei $R_1$ und $R_2$ die oben angegebene Bedeutung haben und R′ bevorzugt $C_1$-$C_3$-Alkyl darstellt, in Gegenwart einer stark basischen Verbindung, wie z.B. einem Alkali- oder Magnesium-alkoholat, -hydrid oder -amid, insbesondere Natriummethylat oder Natriumethylat. Die Umsetzung findet vorzugsweise in einem inerten Lösungsmittel, wie z.B. Toluol, Xylol oder Tetrahydrofuran, statt oder in einem Lösungsmittelgemisch, wie z.B. Di-n-butylether/Dimethylformamid, Di-n-butylether/Tetrahydrofuran. Die Reaktionstemperatur liegt zweckmässig bei 5° bis 25°C. Das primäre Reaktionsprodukt ist das Alkalimetall- oder Magnesium-chelat der Verbindung der Formel I, das durch Säuren, wie z.B. Salzsäure, Schwefelsäure oder Ameisensäure, gespalten werden kann. Es wird dann die entsprechende Verbindung der Formel I erhalten. Das Rohprodukt kann anschliessend nach üblichen Methoden (z.B. Umkristallisation oder Destillation) aufgearbeitet werden.

Verbindungen der Formel I, worin $R_2$ lineares oder verzweigtes $C_2$-$C_5$Hydroxyalkyl bedeutet, werden vorzugsweise durch Umsetzung einer Verbindung der Formel II mit einem Lacton der Formel IIIA,

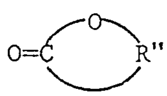

(IIIA)

worin R″ lineares oder verzweigtes $C_2$-$C_5$-Alkylen bedeutet, hergestellt.

Die oben beschriebenen Umsetzungen sind in der Literatur als "CLAISEN-Kondensation" bekannt.

Die Verbindungen der Formel I, worin die in den Resten $R_1$ und $R_2$ enthaltenen OH Gruppen durch $C_2$-$C_{12}$-Alkanoyloxy ersetzt sind, können mit Hilfe von dem Fachmann bekannten Veresterungsreaktionen hergestellt werden.

Die Ausgangsprodukte sind im Handel erhältlich oder können in Analogie zu bekannten Verfahren hergestellt werden.

Die nachfolgenden Beispiele erläutern die Erfindung weiter. Teile- und Prozentangaben beziehen sich darin, soweit nichts anderes angegeben ist, auf das Gewicht.

<u>Beispiel 1:</u> Herstellung von 1-Phenyl-8-hydroxyoctan-1,3-dion.

Von einem Gemisch aus 90 g Natriummethylatlösung (30%ig in Methanol) und 150 ml Di-n-butylether werden bei Normaldruck unter Schutzgas ($N_2$) 75 g eines Lösungsmittelgemisches abdestilliert. Der Rückstand wird auf 0°C abgekühlt und bei gleicher Temperatur werden 60 g Acetophenon und 100 ml Tetrahydrofuran eingetropft. Es wird nachgerührt, bis eine klare Lösung vorliegt. Anschliessend werden 62,8 g ε-Caprolacton innerhalb von 45 Minuten bei ca. 5°C zugetropft. Das Reaktionsgemisch wird 30 Minuten bei gleicher Temperatur und anschliessend bei 20°C nachgerührt. Nach beendeter Reaktion wird das Reaktionsgemisch mit 300 ml Eiswasser hydrolysiert und die organische Phase mit 100 ml Wasser gewaschen.

Die vereinigten wässrigen Phasen werden mit 50%iger Schwefelsäure auf pH 6 eingestellt und diese Lösung wird dreimal mit je 70 ml Diethylether extrahiert. Die vereinigten Extrakte werden über $Na_2SO_4$ getrocknet und am Rotationsverdampfer bis zum Rückstand eingeengt, der als gelblich kristalline Masse vorliegt. Die Ausbeute beträgt 69,6 g (= 59,4 % der Theorie). Nach Umkristallisation aus einem Di-n-butylether/Toluol Gemisch besitzt das Produkt einen Schmelzpunkt von 51-52°C.

Beispiele 2-4:
Die in Tabelle 1 angegebenen Verbindungen werden in Analogie zu dem in Beispiel 1 beschriebenen Verfahren hergestellt.

Tabelle 1:

| Bsp. | Verbindung | Smp./Kp. |
|---|---|---|
| 2 | 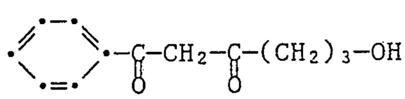 | Smp. 45°C |
| 3 | 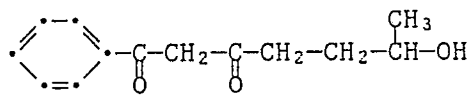 | Smp. 44°C |
| 4 | 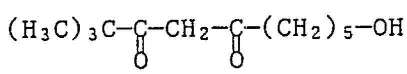 | Kp. 99°C (0,3 bar) |

Beispiel 5: Herstellung von 1-(2′-Hydroxyphenyl)butan-1,3-dion.
Die Verbindung wird in Analogie zu dem von G. Wittig in "Berichte 58, 19 (1925)" beschriebenen Verfahren hergestellt.
Schmelzpunkt: 90°C

Beispiel 6: Herstellung von 1-Phenyl-8-acetyloxyoctan-1,3-dion.
In einem 100 ml-Zweihalskolben mit Magnetrührer und Thermometer werden 6,1 g (0,06 Mol) Essigsäureanhydrid und 1 Tropfen konzentrierte Schwefelsäure vorgelegt. 11,7 g (0,05 Mol) der Verbindung aus Beispiel 1 werden unter Rühren protionsweise zugegeben. Das Gemisch wird 30 Minuten nachgerührt und anschliessend mit 100 ml Diethylether verdünnt. Dann wird das Gemisch einmal mit 100 ml Wasser, zweimal mit 100 ml Bicarbonat und wiederum einmal mit 100 ml Wasser gewaschen, getrocknet und am Rotationsverdampfer bis zum Rückstand eingeengt, der destillativ gereinigt wird. Das erhaltene Produkt besitzt einen Schmelzpunkt von 23-24°C.

Beispiel 7: Herstellung von 1-Phenyl-6-acetyloxyhexan-1,3-dion.
In einem 100 ml-Zweihalskolben mit Magnetrührer, Rückflusskühler und Thermometer werden 8,2 g (0,08 Mol) Essigsäureanhydrid und 1 Tropfen konzentrierte Schwefelsäure vorgelegt. 15,5 g (0,075 Mol) der Verbindung aus Beispiel 2 werden portionsweise unter Rühren zugegeben, wobei das Reaktionsgemisch gekühlt wird, da die Reaktion leicht exotherm abläuft. Das erhaltene Gemisch wird über Nacht nachgerührt. Dann werden 100 ml einer Mischung aus Diethylether und Wasser eingerührt. Die etherische Phase wird abgetrennt, getrocknet und bis zum Rückstand eingeengt, der aus Ethanol/Petrolether umkristallisiert wird. Nach fraktionierter Destillation wird der Rückstand aus Toluol/Petrolether umkristallisiert. Das erhaltene Produkt besitzt einen Schmelzpunkt von 48-49°C.

Beispiel 8: Herstellung von 1-Phenyl-8-undecanoyloxyoctan-1,3-dion.
In einem 250 ml-Vierhalskolben mit Wasserabscheider, KPG-Rührer, Thermometer, Rückflusskühler und Blasenzähler werden 11,7 g (0,05 Mol) der Verbindung aus Beispiel 1, 10,0 g (0,05 Mol) Laurinsäure und 100 ml Toluol 1 Stunde am Rückfluss erwärmt. Es kann kein Reaktionswasser nachgewiesen werden, d.h. es ist keine Reaktion eingetreten. Nach Zugabe von 0,3 g Paratoluolsulfonsäure wird das Reaktionsgemisch weiter am Rückfluss erwärmt. Nach 3 Stunden sind 0,6 ml Wasser (berechnet: 0,9 ml) abgeschieden. Das Reaktionsgemisch wird abgekühlt und mit 50 ml Bicarbonat und 50 ml Wasser gewaschen. Die organische Phase wird getrocknet und bis zum Rückstand eingeengt, der aus einer Mischung von 140 ml i-Propanol und 10 ml $H_2O$ umkristallisiert wird. Das erhaltene Produkt besitzt einen Schmelzpunkt von 37-38°C

Beispiel 9: Hitzestabilität von Polyvinylchlorid.

a) statischer Hitzetest

Eine Trockenmischung bestehend aus 100 Teilen S-PVC (K-Wert 70), 17 Teilen Dioctylphthalat, 3 Teilen epoxidiertem Sojabohnenöl, 0,33 Teilen Zinkoleat, 0,53 Teilen Barium-p-tert-butylbenzoat, 0,7 Teilen Di-isodecyl-phenylphosphit, 0,44 Teilen ®SHELL SOL A (aromatisches Kohlenwasserstoff-Gemisch) und 0,2 Teilen des in Tabelle 2 angegebenen Stabilisators wird auf einem Mischwalzwerk 5 Minuten bei 180° C gewalzt. Vom gebildeten 0,3 mm dicken Walzfell werden Folienmuster in einem Testofen (®Mathis Thermotester Typ LTF-ST) bei 180° C thermisch belastet und im angegebenen Zeitintervall an einem Prüfmuster der "Yellowness Index" (YI) nach ASTM D 1925 bestimmt. Die Ergebnisse sind in Tabelle 2 zusammengefasst.

Tabelle 2

| Stabilisator | YI nach Belastungszeit in Minuten | | | | | | |
|---|---|---|---|---|---|---|---|
| | 0 | 5 | 10 | 15 | 20 | 25 | 30 |
| ohne | 9,5 | 12,0 | 15,1 | 19,5 | 26,0 | 28,8 | 29,3 |
| Verbindung aus Beispiel 1 | 0,8 | 1,0 | 0,9 | 1,8 | 2,0 | 2,3 | 2,7 |

b) dynamischer Hitzetest

Eine Trockenmischung bestehend aus 100 Teilen S-PVC, 1 Teil Polyacrylat (Fliesshilfe), 10 Teilen Methylmethacrylat-Butadien-Styrol-Harz (Schlagzähzusatz), 0,1 Teil Polyethylenwachs, 1 Teil ®Loxiol GH4 (Gleitmittel), 1,43 Teilen Di-n-octyl-zinn-dipropanoat, 0,07 Teilen 2,6-Di-tert-butyl-4-methylphenol, 1 Teil epoxidiertem Sajabohnenöl, 0,1 Teil Zn-bis(ethylhexanoat) und 0,5 Teilen der in Tabelle 3 angegebenen Verbindung wird auf einem Mischwalzwerk bei 190°C gewalzt. In regelmässigen Zeitintervallen werden Folienstücke entnommen, deren Vergilbung nach ASTM D 1925 bestimmt wird. Die Ergebnisse sind in Tabelle 3 angegeben.

Tabelle 3

| Stabilisator | YI nach Belastungszeit in Minuten | | |
|---|---|---|---|
| | 5 | 10 | 15 |
| ohne | 22,8 | 52,7 | 82,4 |
| Verbindung aus Beispiel 1 | 3,3 | 5,6 | 9,5 |

**Patentansprüche**

1. Zusammensetzung enthaltend a) ein chlorhaltiges Polymerisat und b) mindestens eine Verbindung der Formel I, $R_1-\underset{\underset{O}{\|}}{C}-CH_2-\underset{\underset{O}{\|}}{C}-R_2$ (I)

worin $R_1$ $C_1$-$C_{10}$-Alkyl, $C_2$-$C_{10}$-Hydroxyalkyl, Phenyl, Hydroxyphenyl, $C_7$-$C_{10}$-Phenylalkyl oder am Phenylring durch eine OH-Gruppe substituiertes $C_7$-$C_{10}$-Phenylalkyl ist und $R_2$ $C_2$-$C_{10}$-Hydroxyalkyl, Hydroxyphenyl oder am Phenylring durch eine OH-Gruppe substituiertes $C_7$-$C_{10}$-Phenylalkyl darstellt, mit der Bedingung, dass $R_2$ verschieden von Hydroxyphenyl ist, wenn $R_1$ Phenyl oder Hydroxyphenyl bedeutet, und/oder mindestens eine Verbindung der Formel I, worin die in den Resten $R_1$ und $R_2$ enthaltenen OH Gruppen durch $C_2$-$C_{12}$-Alkanoyloxy ersetzt sind.

2. Zusammensetzung gemäss Anspruch 1, enthaltend als Komponente b) mindestens eine Verbindung der Formel I, worin $R_1$ $C_1$-$C_{10}$-Alkyl, $C_2$-$C_{10}$-Hydroxyalkyl, Phenyl, Hydroxyphenyl, $C_7$-$C_{10}$-Phenylalkyl oder am Phenylring durch eine OH-Gruppe substituiertes $C_7$-$C_{10}$-Phenylalkyl ist und $R_2$ $C_2$-$C_{10}$-Hydroxyalkyl, Hydroxyphenyl oder am Phenylring durch eine OH-Gruppe substituiertes $C_7$-$C_{10}$-Phenylalkyl darstellt, mit der Bedingung, dass $R_2$ verschieden von Hydroxyphenyl ist, wenn $R_1$ Phenyl oder Hydroxyphenyl bedeutet.

3. Zusammensetzung gemäss Anspruch 2, worin $R_1$ $C_1$-$C_{10}$-Alkyl, $C_2$-$C_{10}$-Hydroxyalkyl oder Phenyl bedeutet und $R_2$ $C_2$-$C_{10}$-Hydroxyalkyl oder Hydroxyphenyl ist.

4. Zusammensetzung gemäss Anspruch 2, worin $R_1$ $C_1$-$C_4$-Alkyl oder Phenyl bedeutet und $R_2$ $C_2$-$C_7$-Hydroxyalkyl oder Hydroxyphenyl ist.

5. Zusammensetzung gemäss Anspruch 2, worin $R_2$ eine Gruppe $-(CH_2)_n-OH$ bedeutet, wobei n eine ganze Zahl von 2 bis 7 darstellt.

6. Zusammensetzung gemäss Anspruch 2, worin $R_1$ Methyl, Butyl oder Phenyl ist und $R_2$ 2-Hydroxyethyl, 5-Hydroxypentyl, 6-Hydroxyhexyl, 7-Hydroxyheptyl oder ortho-Hydroxyphenyl darstellt.

7. Zusammensetzung gemäss Anspruch 2, worin die Verbindung der Formel I
1-Phenyl-8-hydroxyoctan-1,3-dion,
1-t-Butyl-8-hydroxyoctan-1,3-dion
oder
1-(o-Hydroxyphenyl)butan-1,3-dion
ist.

8. Zusammensetzung gemäss Anspruch 2, worin die Verbindung der Formel I
1-Phenyl-8-hydroxyoctan-1,3-dion
ist.

9. Zusammensetzung gemäss Anspruch 2, worin das chlorhaltige Polymerisat Polyvinylchlorid ist.

10. Zusammensetzung gemäss Anspruch 2, enthaltend zusätzlich eine Epoxiverbindung und mindestens ein Me(II)-Carboxylat und/oder Me(II)-Phenolat, wobei Me(II) Ba, Ca, Mg, Cd oder Zn bedeutet.

11. Zusammensetzung gemäss Anspruch 2, enthaltend zusätzlich epoxidiertes Sojabohnenöl und mindestens ein Me(II)-Carboxylat, wobei Me(II) Ba, Ca, Mg oder Zn bedeutet.

12. Zusammensetzung gemäss Anspruch 10, enthaltend zusätzlich ein Phosphit.

13. Zusammensetzung gemäss Anspruch 2, enthaltend zusätzlich eine Organozinnverbindung.

14. Verwendung von Verbindungen der Formel I,

$$R_1-\underset{\underset{O}{\|}}{C}-CH_2-\underset{\underset{O}{\|}}{C}-R_2 \quad (I)$$

worin $R_1$ $C_1$-$C_{10}$-Alkyl, $C_2$-$C_{10}$-Hydroxyalkyl, Phenyl, Hydroxyphenyl, $C_7$-$C_{10}$-Phenylalkyl oder am Phenylring durch eine OH-Gruppe substituiertes $C_7$-$C_{10}$-Phenylalkyl ist und $R_2$ $C_2$-$C_{10}$-Hydroxyalkyl, Hydroxyphenyl oder am Phenylring durch eine OH-Gruppe substituiertes $C_7$-$C_{10}$-Phenylalkyl darstellt, mit der Bedingung, dass $R_2$ verschieden von Hydroxyphenyl ist, wenn $R_1$ Phenyl oder Hydroxyphenyl bedeutet, zum Stabilisieren von chlorhaltigen Polymerisaten gegen thermischen Abbau.

15. Verbindungen der Formel IA,

$$R-\underset{\underset{O}{\|}}{C}-CH_2-\underset{\underset{O}{\|}}{C}-(CH_2)_5-OH \quad (IA)$$

worin R $C_1$-$C_{10}$-Alkyl, $C_2$-$C_{10}$-Hydroxyalkyl, Phenyl, Hydroxyphenyl, $C_7$-$C_{10}$-Phenylalkyl oder am Phenylring durch eine OH-Gruppe substituiertes $C_7$-$C_{10}$-Phenylalkyl ist.

16.Verbindungen gemäss Anspruch 15, worin R $C_1$-$C_{10}$-Alkyl, Phenyl oder $C_7$-$C_{10}$-Phenylalkyl ist.

17.Verbindungen gemäss Anspruch 15, worin R $C_1$-$C_4$-Alkyl oder Phenyl bedeutet.

18. Die Verbindungen
1-Phenyl-8-hydroxyoctan-1,3-dion
und
1-t-Butyl-8-hydroxyoctan-1,3-dion
gemäss Anspruch 15.

Europäisches Patentamt

# EUROPÄISCHER RECHERCHENBERICHT

Nummer der Anmeldung

EP 89 81 0399

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | US-A-4 381 360 (LEISTNER et al.)<br>* Spalte 25, Punkt 27 *<br>----- | 1 | C 08 K 5/07<br>C 08 L 57/08<br>C 07 C 49/12 |

RECHERCHIERTE SACHGEBIETE (Int. Cl.4)

C 07 C
C 08 K
C 08 L

Der vorliegende Recherchenbericht wurde für alle Patentansprüche erstellt

| Recherchenort | Abschlußdatum der Recherche | Prüfer |
|---|---|---|
| DEN HAAG | 30-08-1989 | SCHUELER D.H.H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)